# NEUE EUROPÄISCHE PATENTSCHRIFT

(11) **EP 1 035 825 B2**
(45) Veröffentlichungstag und Bekanntmachung des Hinweises auf die Entscheidung über den Einspruch: **24.09.2008**
(45) Hinweis auf die Patenterteilung: 25.09.2002
(21) Anmeldenummer: 98959895.8
(22) Anmeldetag: 28.11.1998
(51) Int. Cl.: A61K 8/04, A61K 8/34, A61K 8/36, A61K 8/39, A61K 8/45, A61Q 5/10

(54) **HAARFÄRBEMITTEL-ZUBEREITUNG**
HAIR COLORANT PREPARATION
PREPARATION DE COLORANT CAPILLAIRE

(30) Priorität: 08.12.1997 DE 19754281
(43) Veröffentlichungstag der Anmeldung: 20.09.2000
(73) Patentinhaber: Henkel AG & Co. KGaA, 40589 Düsseldorf (DE)
(72) Erfinder: HÖFFKES, Horst, D-40595 Düsseldorf (DE); MEINIGKE, Bernd, D-51381 Leverkusen (DE); SCHETTIGER, Norbert, D-40723 Hilden (DE); GOUTSIS, Konstantin, D-41470 Neuss (DE)
(86) Internationale Anmeldenummer: PCT/EP1998/007702
(87) Internationale Veröffentlichungsnummer: WO 1999/029285

(56) Entgegenhaltungen:
- EP-A- 0 188 216
- EP-A- 0 351 645
- DE-A1- 3 834 142
- DE-A1- 4 022 848
- DE-A1- 4 103 292
- DE-A1- 4 421 032
- DE-A1- 19 614 303
- DE-C1- 4 301 663
- DE-T2- 69 100 880
- US-A- 5 021 066
- US-A- 5 525 123
- US-A1- 4 698 065
- Bd. 2 1989 Artikel K.SCHRADER: 'Grundlagen und Rezepturen der Kosmetika
- CHARLES ZVIAK: 'The Science of Hair Care', 1986, MARCEL DEKKER, INC, NEW YORK, ISBN 0824773780 Seite 70
- KARLHEINZ SCHRADER: 'Grundlagen und Rezepturen der Kosmetica', 1989, HOTHIG BUCH VERLAG, HEIDELBERG, ISBN 3778514911 Seiten 600 - 601
- DR.JORGEN FALBE,DR.MANFRED REGITZ: 'Römpp Chemie Lexikon', Bd. 9, 1989, GEORG THIEME VERLAG, STUTTGART, ISBN 3137346096 Seite 628
- 'International Cosmetic Ingredient Handbook', 1995, THE COSMETIC, TOILETRY, AND FRAGANCE ASSOCIATION Seiten 962 - 964

## Beschreibung

Die Erfindung betrifft Haarfärbemittel-Zubereitungen, bestehend aus einer fließfähigen wäßrigen Zubereitung von Oxidationsfarbstoffvorprodukten (A) und einer fließfähigen, wäßrigen Oxidationsmittelzubereitung (B), die unmittelbar vor dem Aufbringen auf das Haar zu einem gelförmigen Färbeansatz vermischt werden, und die nach dem Vermischen als strukturviskoses, fließfähiges Gel vorliegen.

Als Träger für Oxidationshaarfärbemittel werden überwiegend O/W-Cremes oder Gele verwendet. Die Zubereitungen von Oxidationsfarbstoffvorprodukten sollen nach Zugabe des Oxidationsmittels, meist einer wäßrigen Wasserstoffperoxid-Zubereitung, eine cremige oder viskos gelförmige Konsistenz aufweisen, die ein leichtes Auftragen und Verteilen auf dem Haar, z.B. mit einem Pinsel, und eine gewisse Haftung auf dem Haar ermöglicht, ohne daß das Färbemittel vom Haar abläuft und Kopfhaut oder Gesicht benetzt.

Oxidationsfarbstoffzubereitungen vom Gel-Typ weisen als Träger üblicherweise Seifen im Gemisch mit Wasser und niederen Alkoholen oder Glycolen auf. Solche Zubereitungen ergeben nach dem Vermischen mit wäßrigen Oxidationsmittel-Lösungen viskose, gelförmige Färbeansätze. Nachteilig ist die Bildung von Kalkseifen beim Ausspülen des Färbemittels mit hartem Wasser. Ein weiterer Nachteil besteht darin, daß bei einem hohen Seifenanteil die Farbstoff-Zubereitung zu viskos wird, bei einem niedrigeren Seifenanteil aber der Färbeansatz nicht die erforderliche Viskosität erreicht. Auch die Mitverwendung eines synthetischen Tensids mit einer die Kalkseife dispergierenden Wirkung stört die Ausbildung der fließfähigen Gelstruktur des Färbeansatzes.

Aus der DE 40 22 848 A1 ist eine Haarfärbemittelzubereitung bekannt, die sich zur Aufgabe gestellt hat, die oben genannten Nachteile zu vermeiden. Das dort offenbarte Trägersystem enthält Seife, Polyole, synthetische Tenside sowie Anlagerungsprodukte von Ethylenoxid an lineare Fettalkohole und/ oder lineare Fettalkylamine. Dieses Trägersystem zeigt jedoch bei der Applikation noch Nachteile bezüglich der avivierenden Wirkung, gleichzeitig sind die Viskositäten relativ hoch.

Die Aufgabe der vorliegenden Erfindung war es daher, ein für die Oxidationsfärbemittel geeignetes Trägersystem zu finden, das die zuvor genannten Probleme bei gleichzeitiger guter Avivage nicht oder in erheblich geringerem Ausmaß aufweist.

Es wurde gefunden, daß Haarfärbemittel, bestehend aus einer fließfähigen, wäßrigen Zubereitung von Oxidationsfarbstoffvorprodukten (A) und einer fließfähigen, wäßrigen Oxidationsmittelzubereitung (B), die unmittelbar vor dem Aufbringen auf das Haar miteinander im Gewichtsverhältnis A:B = 1:2 bis 2:1 zu einem gelförmigen Färbeansatz vermischt werden, dann besonders gute Anwendungseigenschaften aufweisen, wenn die Zubereitung von Oxidationsfarbstoffvorprodukten (A) als Trägerkomponenten
- 6,0 bis 15 Gew.-%: technischer Fettalkoholmischungen aus gesättigten oder ungesättigten, linearen oder verzweigten Alkoholen mit 12 bis 18 Kohlenstoffatomen, ausgewählt aus Kokos-, oder Palmkernalkohol,
- 0,1 bis 20 Gew.-%: eines niedermolekularen wasserlöslichen Alkohols,
- 0,1 bis 15 Gew.-%: einer flüssigen Fettsäure mit 16 bis 22 C-Atomen in Form einer wasserlöslichen Seife,
- 0,1 bis 20 Gew.-%: eines Anlagerungsproduktes von 1 bis 5 Mol Ethylenoxid an einen linearen Fettalkohol mit 8 bis 22 C-Atomen und möglicherweise
- 0,1 bis 15 Gew.-%: eines Amins gemäß Formel (I), 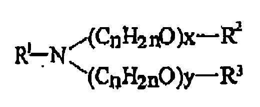 in der R¹ für einen gesättigten oder ungesättigten Alkylrest mit 8 bis 22 C-Atomen, R² und R³ unabhängig voneinander für Wasserstoff oder eine Acyl-Gruppe R⁴CO stehen, bei der wiederum R⁴ für eine C₁-C₂₁-Alkyl- oder C₂-C₂₁- Alkenylgruppe steht n ist gleich 2 oder 3, x und y stehen unabhängig voneinander für Zahlen von 0 bis 5, mit der Maßgabe, daß die Summe aus x und y 2 bis 6 beträgt,
enthalten und die Oxidationsmittelzubereitung (B)
- 3 bis 12 Gew.-%: Wasserstoffperoxid
- 0,1 bis 5 Gew.-%: eines wasserlöslichen, synthetischen Tensids und
- 1 bis 5 Gew.-%: eines dispergierten Acrylsäure- und/oder Methacrylsäure-Polymerisats oder -Copolymerisats enthält.

In einer bevorzugten Ausführungsform können weiterhin
- 0,1 bis 20 Gew.-%: eines Polyols und/oder
- 0,5 bis 10 Gew.-%: eines wasserlöslichen, synthetischen Tensids und/ oder
- 0,1 bis 10 Gew.-%: einer Verbindung der Formel (II)

R⁵-(OC₂H₄)ₓ-A-(C₂H₄O)_{y}-R⁶ (II)

in der R⁵ und R⁶ lineare Alkyl- oder Alkenylgruppen mit 12 bis 22 C-Atomen, x und y = 0 oder 1 und A ein Sauerstoffatom oder eine Gruppe HO―C₂H₄-N〈 ist
enthalten sein.

Bei den erfindungsgemäß einzusetzenden gesättigten oder ungesättigten, linearen oder verzweigten **Alkoholen** mit 8 bis 36 C-Atomen handelt es sich vorzugsweise um Fettalkohole und/ oder Guerbetalkohole.

Unter **Fettalkoholen** sind primäre aliphatische Alkohole der Formel **(III)** zu verstehen,

R⁷OH (III)

in der R⁷ für einen aliphatischen, linearen oder verzweigten Kohlenwasserstoffrest mit 8 bis 22 Kohlenstoffatomen, der gesättigt ist oder bis zu 3 Doppelbindungen enthalten kann, steht.

Typische Beispiele sind 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestem auf Basis von Fetten und Ölen oder Aldehyden aus der Roelen'schen Oxosynthese sowie als Monomerfraktion bei der Dimerisierung von ungesättigten Fettalkoholen anfallen.

Unter **Guerbetalkoholen** sind Alkohole zu verstehen, die durch alkalische Kondensation von Alkoholen zu höhermolekularen, verzweigten Iso-Alkoholen hergestellt werden. Diese Umsetzung wurde erstmals von Guerbet 1899 veröffentlicht. Machemer stellte 1952 wesentliche Schritte der Reaktion dar (Angewandte Chemie 64 (1952) 213 - 20):

Neben der Dehydrierung zum Keton, bei der Wasserstoff abgespalten wind, und der Aldolkondensation ist die Crotonisierung, bei der Wasser abgespalten wird, ein wichtiger Schritt im Reaktionsablauf. Stand der Technik ist eine Reaktionsführung bei Normaldruck und einer Reaktionstemperatur von 240 bis 260 °C. Die so erhaltenen verzweigten Alkohole weiden als Guerbetalkohole bezeichnet. Aus dem Stand der Technik sind inzwischen eine Vielzahl weiterer Verfahren bekannt, gemäß derer man Guerbetalkohole erhalten kann.

Unter **niedermoleknlaren Alkoholen** sind im Sinne der vorliegenden Erfindung mit Wasser mischbare Alkohole mit 1 bis 5 C-Atomen zu verstehen. Vorzugsweise handelt es sich um Ethanol, Propanol und/ oder Isopropanol.

Die zur Seifenbildung geeigneten **Fettsäuren** sind bevorzugt flussige oder niedrigschmelzende ungesättigte lineare C₁₆-C₂₂-Fettsäuren wie Palmitoleinsäure, Ölsäure, Elaidinsäure, Petroselinsäure, Petroselaidinsäure, Gadoleinsäure, Erucasäure, Brassidinsäure sowie Gemische dieser Fettsäuren untereinander und gegebenenfalls mit geringeren Anteilen gesättigter linearer Fettsäuren mit 12 bis 22 C-Atomen. Andere ebenfalls geeignete Fettsäuren sind verzweigte Fettsäuren mit 16 bis 22 C-Atomen, z.B. die 2-Hexyldecansäure, Isostearinsäure und die 2-Octyldodecansäure.

Zur Überführung der Fettsäuren in wasserlösliche Seifen eignen sich Alkalihydroxide und Alkalicarbonate, Ammoniak, Mono-, Di- und Trialkanolamine mit 2 bis 4 C-Atomen in der Alkanolgruppe sowie alkalische Aminosäuren wie beispielsweise Arginin, Omithin, Lysin und/ oder Histidin.

Als **Polyole** eignen sich z.B. Ethylenglycol, 1,2-Propylenglycol, 1,3-Propylenglycol, Glycerin, Erythrit, Trimethylolpropan, Diethylenglycol und Dipropylenglycol. 1,2-Propylenglycol ist bevorzugt.

Als **wasserlösliche synthetische Tenside** eignen sich bevorzugt anionische, amphotere, zwitterionische und nichtionische Tenside mit guter Wasserlöslichkeit und mit gutem Kalkseifendispergiervermögen. Solche Tenside weisen in der Regel eine lipophile lineare Alkyl- oder Acylgruppe mit 12 bis 18 C-Atomen und eine stark dissoziierte ionische Gruppe oder eine nichtionische wasserlöslichmachende Polyethergruppe auf. Geeignet sind z.B. Schwefelsäurehalbestersalze von linearen Fettalkoholen mit 12 bis 18 C-Atomen oder von Fettalkoholpolyglycolethern mit 12 bis 16 C-Atomen in der Alkylgruppe und 1 bis 10 Glycolethergruppen. Weitere geeignete Aniontenside sind z.B. lineare Alkansulfonate und α-Olefinsulfonate mit 12 bis 18 C-Atomen. Geeignete nichtionogene Tenside sind z.B. die Anlagerungsprodukte von 7 bis 30 Mol Ethylenoxid an lineare Fettalkohole mit 12 bis 18 C-Atomen, an Fettsäuren mit 12 bis 18 C-Atomen sowie an Fettsäuremonoglyceride und an Fettsäure-sorbitanmonoester. Bevorzugt geeignete nichtionogene Tenside sind die Fettalkylaminoxide und insbesondere die Fettalkylglykoside, vorzugsweise Fettalkylglucoside. Die Fettalkylgruppe kann in den genannten Produkten 12 bis 18 C-Atome aufweisen. Besonders bevorzugt im Sinne der vorliegenden Erfindung sind auch amphotere Tenside wie beispielsweise N-Fettalkyl-dimethyl-glycin oder N-Fettalkylaminopropionsäure und/ oder zwitterionische Tenside, z.B. N-Fettalkyldimethylammoniumglycinat oder N-Fettacylaminopropyldimethylglycinat. Weiterhin bevorzugt werden Kationtenside wie wie quartäre Ammoniumverbindungen (QAV) insbesondere quaternierte Trialkylammoniumverbindungen mit Alkylresten einer Kettenlänge von C8 bis C22.

Es hat sich gezeigt, daß durch den Zusatz weiterer, begrenzt wasserlöslicher, nichtionogener Tenside eine Verdickung der Oxidationsfärbemittel-Zubereitung und insbesondere des unmittelbar vor der Anwendung hergestellten Färbeansatzes erreicht wird.

Als Anlagerungsprodukte von 1 bis 5 Mol Ethylenoxid an linearen **Fettalkohol** mit 12 bis 22 C-Atomen eignen sich alle nach den bekannten technischen Oxethylierungsverfahren erhältlichen Addukte. Bevorzugt sind die Anlagerungsprodukte, die nur wenig freien Fettalkohol enthalten und eine eingeengte Homologenverteilung aufweisen (sogenannte "narrow range ethoxylates"), wie sie z.B. nach dem in DE 38 43 713 A1 beschriebenen Verfahren zugänglich sind.

Als Anlagerungsprodukte von 1 bis 4 Mol Ethylenoxid an ein lineares **Fettalkylamin** mit 12 bis 22 C-Atomen eignen sich alle nach bekannten technischen Verfahren zugänglichen Addukte, die auch im Handel erhältlich sind. Besonders geeignet ist das Anlagerungsprodukt von 2 Mol Ethylenoxid an ein C₁₂-C₁₈-Kokosalkylamin.

Geeignete Verbindungen der Formel

R⁵-(OC₂H₄)ₓ-A-(C₂H₄O)_{y}-R⁶ (II)

in welcher A ein Sauerstoffatom ist, sind Dialkylether mit 12 bis 18 C-Atomen in den Alkylgruppen. Solche Produkte sind literaturbekannt. Noch besser geeignet sind die Produkte der Formel II, in welchen x oder y oder beide = 1 sind. Solche Di-alkyloxethylether lassen sich durch literaturbekannte Veretherungsverfahren aus Fettalkoholen und Fettalkyloxyethanolen herstellen. Die Produkte der Formel II, in welchen A eine Gruppe HO-C₂H₄-N〈 ist, lassen sich z.B. aus Triethanolamin durch O-Alkylierung mit 2 Mol eines Schwefelsäurehalbestersalzes eines C₁₂-C₂₂-Fettalkohols nach dem in DE 35 04 242 beschriebenen Verfahren zur Herstellung von Etheraminen gewinnen.

Besonders bevorzugte Verbindungen der Formel II sind z.B. Di-cetyl-stearylether, Dicetyl-stearyl-dioxyethyl-ether und N,N-Bis-(2-cetyl-/stearyl-oxyethyl)-aminoethanol.

Während mit Anlagerungsprodukten von 1 bis 5 Mol Ethylenoxid an einen linearen Fettalkohol die erforderliche Verdickung meist allein durch diese Komponente erreicht wird, kann es bei Einsatz der Anlagerungsprodukte von 1 bis 4 Mol Ethylenoxid an ein lineares Fettalkylamin vorteilhaft sein, diese in Kombination mit 1 bis 10 Gew.-% einer Verbindung der Formel II zu verwenden. Es wird dabei angestrebt, daß die Zubereitung von Oxidationsfarbstoffvorprodukten (A) eine Viskosität von höchstens 1 Pas (20 °C) und der Färbeansatz, der durch Vermischen von (A) mit der Oxidationsmittelzubereitung (B) entsteht, eine Viskosität von wenigstens 10 Pas (20 °C) aufweist (Viskositätsmessung mit dem Rotationsviskosimeter Brookfield, Typ RTV, Spindel 4, 4 UpM).

Darüber hinaus sind in der Zubereitung von Oxidationsfarbstoffvorprodukten (A) natürlich die Oxidationsfarbstoffvorprodukte, die in Gegenwart von Oxidationsmitteln den Farbstoff ausbilden, sowie gegebenenfalls auch Direktfarbstoffe enthalten. Als Oxidationsfarbstoffvorprodukte eignen sich z.B. die bekannten Farbbasen bzw. Entwicklerverbindungen und bekannte Modifikatoren bzw. Kupplerverbindungen. Die Oxidationsfarbstoffe bilden sich durch oxidative Kupplung einer oder mehrerer Entwicklerkomponenten untereinander oder mit einer oder mehreren Kupplerkomponenten in Gegenwart eines Oxidationsmittels aus Als **Entwicklerkomponenten** werden üblicherweise primäre aromatische Amine mit einer weiteren, in para- oder ortho-Position befindlichen, freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Typische Beispiele sind p-Phenylendiamin, p-Toluylendiamin, p-Aminophenol, 1-(β-Hydroxyethyl)-2,5-diaminobenzol, N,N-Bis-(2-hydroxy-ethyl)-p-phenylendiamin, 2-(2,5-Diaminophenoxy)-ethanol, 4,5-Diamino-1-(2-hydroxyethyl)-pyrazol, 1-Phenyl-3-carboxyamido-4-amino-pyrazolon-5, 4-Amino-3-methylphenol, 2-Hydroxymethyl-4-aminophenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diamino-pyrimidin, 2-Dimethylamino-4,5,6-triaminopyrimidin, 2-Hydroxyethylaminomethyl-4-amino-phenol, 1,3-Bis-[N-(2-hydroxyethyl)-N-(4-aminophenyl)-amino]-2-propanol sowie 4,4'-Diaminodiphenylamin. Bevorzugte Entwicklerkomponenten im Sinne der vorliegenden Erfindung sind 1,3-Bis-(N-(2-Hydroxyethyl)-N-(4-aminophenyl)-amino)-2-propanol, 6-Chlor-2-aminophenol, 1,10-Bis(2,5-diaminophenyl)-1,4,7,10-tetraoxadecan; besonders bevorzugt werden 6-Hydroxy-2,4,5-triaminopyrimidin, 2-(2,5-Diamino-phenyl)ethanol, 2,6-Dichlor-4-aminophenol, 3-Methyl-4-aminophenol, 2,4,5,6-Tetraamino-pyrimidin, 2-Aminomethyl-4-aminophenol und 2 Hydroxymethyl-4-aminophenol einge-setzt.

Als **Kupplerkomponenten** werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin, 2,5-Dimethylresorcin, 2,6-Dihydroxypyridin, 2,6-Diaminopyridin, 2,6-Dihydroxy-3,4-dimethylpyridin, 2,6-Dihydroxy-4-methylpyridin, 3-Amino-2-methylamino-6-methoxypyridin, 2,6-Bis-(2-hydroxyethylamino)-toluol, 2,4-Diaminophenoxyethanol und 2-Amino-4-hydroxyethylamino-anisol. Im Sinn der vorliegenden Erfindung werden vorzugsweise folgende Kupplersubstanzen eingesetzt: 2,4-Diaminophenoxyethanol, 2-Methylresorcin, 1,3-Bis-(2,4-diaminophenoxy)propan, 2,7-Dihydroxynaphthalin, α-Naphthol, 2,6-Dihydroxy-3,4-dimethylpyridin, 1,5-Dihydroxynaphthalin, 5-Amino-2-methylphenol, 2-Amino-4-(β-hydroxyethylamino)anisol, 3-Amino-2-chlor-6-methyl-phenol und 5-Amino-4-chlor-2-methylphenol. Besonders bevorzugt sind 2,4-Diaminophenoxyethanol, 1,3-Bis-(2,4-diaminophenoxy)propan, 2-Methylresorcin, 2,7-Dihydroxynaphthalin und 2,6-Dihydroxy-3,4-dimethylpyridin.

Darüber hinaus können die Zubereitung von Oxidationsfarbstoffvorprodukten (A) geeignete Zusätze zur Stabilisierung der Farbstoffvorprodukte enthalten; dies sind **Komplexbildner,** z.B. Ethylendiaminotetraessigsäure, Nitrilotriessigsäure, 1-Hydroxyethan-1,1-diphosphonsäure oder andere Organodiphosphonsäuren in Form ihrer Alkalisalze, **Antioxidantien**, wie z.B. Natriumsulfit, Natriumbisulfit, Hydrochinon oder Salze der Thioglycolsäure oder Ascorbinsäure, **Puffersalze,** wie z.B. Ammoniumsulfat, Ammoniumcarbonat, Ammoniumcitrat sowie Ammoniak oder ein Alkanolamin zur Einstellung eines pH-Wertes von 8 bis 10.

In einer bevorzugten Ausführungsform enthalten die erfindungsgemäßen Zubereitungen von Oxidationsfarbstoffvorprodukten (A) **Direktzieher,** darunter sind übliche direktziehende Farbstoffezu verstehen, z.B. aus der Gruppe der Nitrophenylendiamine, Nitroaminophenole, Azofarbstoffe, Anthrachinone oder Indophenole, wie beispielsweise unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen, sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol und 4-N-Ethyl-1,4-bis-(2'-hydroxyethylamino)-2-nitrobenzol-hydrochlorid in einer Menge von 0,01 bis 20 Gew.-%, bezogen auf das gesamte Oxidationshaarfärbemittel. 4-Amino-2-nitro-diphenylamin-2'-carbonsäure, 6-Nitro-1,2,3,4-tetrahydrochinoxalin und HC Red BN sind erfindungsgemäß besonders bevorzugte direktziehende Farbstoffe.

In einer bevorzugte Ausführungsform können auch **Indole** und/oder **Indoline** enthalten sein. Im Sinne der vorliegenden Erfindung besonders bevorzugt sind 6-Hydroxyindol, N-Methyl-6-Hydroxyindol, N-Ethyl-6-Hydroxyindol, N-Propyl-6-Hydroxyindol, N-Butyl-6-Hydroxyindol, 4-Hydroxyindol, N-Methyl-4-Hydroxyindol, N-Ethyl-4-Hydroxyindol, N-Propyl-4-Hydroxyindol, N-Butyl-4-Hydroxyindol, 5,6-Dihydroxyindol, N-Methyl-5,6-Dihydroxyindol, N-Ethyl-5,6-Dihydroxyindol, N-Propyl-5,6-Dihydroxyindol, N-Butyl-5,6-Dihydroxyindol sowie 5,6-Dihydroxyindolin, N-Methyl-5,6-Dihydroxyindolin, N-Ethyl-5,6-Dihydroxyindolin, N-Propyl-5,6-Dihydroxyindolin und N-Butyl-5,6-Dihydroxy-indolin.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Zusätzlich können **Polymere**, beispielsweise zwitterionische und/ oder nichtionische Polymere wie Silikonöle, bevorzugt kationische Polymere wie Polymer JR 400, Merquat 100, Gafquat 734, Gafquat 755, Mirapol A15, Hexadimethrinchlorid (Kondensation von N,N,N',N'-Tetramethylhexamethylendiamin und Trimethylenchlorid) und Polyquaternium-34, enthalten sein.

Insbesondere bevorzugt sind Zubereitungen von Oxidationsfarbstoffvorprodukten (A) die **Proteine und/ oder -derivate** pflanzlicher oder tierischer Herkunft wie beispielsweise Erbsen-, Soja-, Weizen- und Mandelproteinhydrolysat oder Akazienprotein sowie Collagen- oder Keratinhydrolysat enthalten.

Als **Verdicker sind erfindungsgemäß bevorzugt** Xanthan-Gum, Agar-Agar, lineare und vernetzte Polyacrylate, nichtionogene und anionische Cellulosederivate.

Weiterhin können haarkosmetische Hilfsstoffe enthalten sein, insbesondere Bisabolol, Pflanzenextrakte, Vitamine wie vorzugsweise Niacinamid, Tocopherol, Vitamin A, Biotin und Vitamin D.

Die Oxidationsmittelzubereitung (B) enthält
- 3 bis 12 Gew.-%: Wasserstoffperoxid
- 0,1 bis 5 Gew.-%: eines wasserlöslichen, synthetischen Tensids und
- 1 bis 5 Gew.-%: eines dispergierten Acrylsäure- und/oder Methacrylsäure-Polymerisats oder -Copolymerisats
im wäßrigen Träger neben den in solchen Zubereitungen üblichen Stabilisierungshilfsmitteln. Sie kann aber auch im einfachsten Fall allein aus Wasser bestehen, so daß die Oxidation durch den Luftsauerstoff herbeigeführt wird. Aus dem Stand der

Technik sind auch eine Reihe von Katalysatoren, wie beispielsweise Salze des Kupfers, Mangans, Eisens, Cers, Lanthans, Vanadiums, Molybdäns und Wolframs bekannt, die im Fall der Oxidation durch Luftsuerstoff der Reaktionsmischung zugesetzt werden können. Wie aus dem Stand der Technik bekannt gibt es auch noch weitere Möglichkeiten der Oxidation, beispielsweise mit Iodid/ Periodat und/ oder Iodat oder in Kombination von Iodid mit Wasserstoffperoxid, sowie enzymatisch. Im Sinne der vorliegenden Erfindung sind alle aus der Literatur bekannten Methoden der Oxidation geeignet, so daß die Oxidations-mittelzubereitung (B) die entsprechenden Substanzen enthalten kann.
Als wasserlösliche synthetische Tenside können in der Oxidationsmittelzubereitung (B) die bereits für die Zubereitung von Oxidationsfarbstoffvorprodukten (A) genannten anionischen, amphoteren, zwitterionischen und nichtionischen Tenside oder Gemische davon verwendet werden. Bevorzugt werden anionische Tenside, z.B. Schwefelsäurehalbestersalze von linearen Fettalkoholen mit 12 bis 18 C-Atomen oder von Fettalkoholpolyglycolethern mit 12 bis 16 C-Atomen in der Alkylgruppe und 1 bis 10 Glycolethergruppen in Form ihrer Alkali-, Magnesium-, Ammonium- oder Alkanolammoniumsalze eingesetzt.

Die Oxidationsmittelzubereitung (B) enthält außerdem bevorzugt Komplexbildner und Puffersalze zur Einstellung eines pH-Wertes von 2 bis 5. In diesem schwach sauren Medium bleiben die Acrylsäure- und/oder Methacrylsäure-Polymerisat-Dispersionen dünnflüssig und stabil. Beim Vermischen mit der alkalischen Zubereitung von Oxidationsfarbstoffvorprodukten (A), die Ammoniak und Puffersalze zur Einstellung eines pH-Wertes von 8 bis 10 enthält, steigt der pH-Wert der Mischung an und die Carboxylgruppen des Polymerisats oder Copolymerisats werden in die Salzform überführt. Dabei beginnen die Polymerisate sich im wäßrigen Medium zu lösen und die Viskosität der Lösung anzuheben.

Besonders günstig für den Viskositätsaufbau nach dem Vermischen der Zubereitung von Oxidationsfarbstoffvorprodukten (A) und der Oxidationsmittelzubereitung (B) ist der Gehalt an dispergierten Acrylsäure- und/oder Methacrylsäure-Polymerisat oder-Copolymerisat in (B). Solche Dispersionen von Copolymerisaten, z.B. aus wenigstens 10 Gew.-% Acrylsäure-Niedrigalkylester, 25 bis 70 Gew.-% Methacrylsäure und ggf. bis zu 40 Gew.-% eines weiteren Comonomeren, sind z.B. in GB 870 994 beschrieben. Aus DE 11 64 095 sind Mischpolymerisate aus 50 bis 75 Gew.-% Ethylacrylat, 25 bis 35 Gew.-% Acrylsäure und 0 bis 25 Gew.-% anderer Comonomerer bekannt. Geeignete Dispersionen dieser Art sind im Handel erhältlich, z.B. unter der Handelsbezeichnung Latekoll ^{(R)}D (BASF). Besonders gut eignen sich die in DE 34 45 549 beschriebenen Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Die folgenden Beispiele sollen den Gegenstand der Erfindung näher erläutern:

### Beispiele

Folgende Zubereitungen von Oxidationsfarbstoffvorprodukten (A) wurden getestet (Alle Angaben sind in Gew.-%):

**Tabelle 1**

| Rohstoff | Rezepturen | | | | | |
|---|---|---|---|---|---|---|
| | 1 | 2 | 3 | 4 | 5 | 6 |
| Ölsäure | 6,75 | 6,75 | 6,75 | 6,75 | 6,75 | 6,75 |
| Lorol^{R} (technisch)¹ | 6,75 | 6,75 | 6,75 | 6,75 | 6,75 | - |
| Texapon^{R} NSW² | 3,95 | 3,95 | 3,95 | 3,95 | 3,95 | 3,95 |
| Plantasol^{R} W 20³ | 3,0 | 3,0 | 3,0 | 3,0 | 3,0 | 3,0 |
| Dehydol^{R} LS-2⁴ | - | 13,5 | 6,75 | - | - | 13,5 |
| Kokosamin 2 EO | - | - | - | 6,75 | - | - |
| Lowenol ^{R}S 216⁵ | - | - | - | - | 6,75 | - |
| 1,2-Propylenglykol | 6,75 | 6,75 | 6,75 | 6,75 | 6,75 | 6,75 |
| Isopropylalkohol | 15,0 | 15,0 | 15,0 | 15,0 | 15,0 | 15,0 |
| Monoethanolamin | 5,0 | 5,0 | 5,0 | 5,0 | 5,0 | 5,0 |
| Arginin | 1,0 | 1,0 | 1,0 | 1,0 | 1,0 | 1,0 |
| Na-metabisulfit | 0,2 | 0,2 | 0,2 | 0,2 | 0,2 | 0,2 |
| Ascorbinsäure | 0,2 | 0,2 | 0,2 | 0,2 | 0,2 | 0,2 |
| Parfümöl | 0,5 | 0,5 | 0,5 | 0,5 | 0,5 | 0,5 |
| Resorcin | 0,06 | 0,06 | 0,06 | 0,06 | 0,06 | 0,06 |
| p-Aminophenolhydrochlorid | 0,06 | 0,06 | 0,06 | 0,06 | 0,06 | 0,06 |
| p-Toluylendiaminsulfat | 0,05 | 0,05 | 0,05 | 0,05 | 0,05 | 0,05 |
| Wasser | ad 100 | | | | | |

| | | | | | | |
|---|---|---|---|---|---|---|
| ¹C12/C18 Fettalkohol (100 Gew.-% Aktivsubstanz) | | | | | | |
| ²C12/C14 Fettalkohol-2EO-sulfat Natrium-Salz (25 Gew.-% Aktivsubstanz in wäßriger Lösung) | | | | | | |
| ³Weizenproteinhydrolysat (20 Gew.-% Aktivsubstanz in wäßriger Lösung) | | | | | | |
| ⁴C12/C14 Fettalkohol-2EO (100 Gew.-% Aktivsubstanz) | | | | | | |
| ⁵Bis(2-hydroxyethyl)sojaalkylamindioleat (97 Gew.-% Aktivsubstanz) | | | | | | |

Die in der Tabelle 1 aufgeführten Zubereitungen von Oxidationsfarbstoffvorprodukten (A), Rezepturen 1 bis 5, wurden anschließend mit der folgend (Tabelle 2) aufgeführten Oxidationsmittelzubereitung (B), Rezeptur 7, im Verhältnis 4:5 gemischt. Danach wurde die Viskosität der entstandenen Gele bestimmt.

**Tabelle 2**

| Rohstoff | Rezeptur 7 Anteil in Gew.-% |
|---|---|
| Wasserstoffperoxid | 5,0 |
| Texapon^{R} NSW² | 2,0 |
| Acrysol ^{R}33⁶ | 5,5 |
| Turpinal^{R} SL⁷ | 1,5 |
| Ammoniak 25 gew.-%ige wäßrige Lsg. | 0,65 |
| Wasser | ad 100 |

| | |
|---|---|
| ⁶Acrylat Copolymer (30 Gew.-% Aktivsubstanz) | |
| ⁷Acetophosphonsäure (60 Gew.-% Aktivsubstanz) | |

Die Messung der Viskosität erfolgte bei 20 °C mit einem Brookfield Rotationsviskosimeter, Type RTV, Spindel 4,4 Umdrehungen pro Minute. Die Ergebnisse sind in Tabelle 3 dargestellt:

**Tabelle 3**

| Rezeptur | Viskosität [mPas] |
|---|---|
| 1+7 | 1975 |
| 2+7 | 18000 |
| 3+7 | 11250 |
| 4+7 | 10500 |
| 5+7 | 12500 |

Zur Ermittlung der avivierenden Eigenschaften der erfindungsgemäßen Rezepturen wurden Rezeptur 2 und 6 verglichen. Dazu wurden beide Rezepturen wie zuvor beschrieben mit Rezeptur 7 gemischt und anschließend 4g der jeweiligen Mischung auf je eine Haarsträhne (2g) aufgetragen und nach 30 minütiger Einwirkung die Kämmbarkeit subjektiv bewertet (mit einem feinzinkigen, gesägten Hartgummikamm). Die Ergebnisse sind Tabelle 4 zu entnehmen.

**Tabelle 4**

| Rezeptur | Kämmbarkeit* |
|---|---|
| Haarsträhne vor der Färbung | 5 |
| Nach Behandlung mit 2+7 | 2 |
| Nach Behandlung mit 6+7 | 4 |

| | |
|---|---|
| * Die Bewertung der Kämmbarkeit erfolgte subjektiv anhand einer Notenskala von 1= sehr gut bis 5 = schlecht. | |

## Patentansprüche

1. Haarfärbemittel, bestehend aus einer fließfähigen Zubereitung von Oxidationsfarbstoffvorprodukten (A) und einer fließfähigen, wäßrigen Oxidationsmittelzubereitung (B), die unmittelbar vor dem Aufbringen auf das Haar miteinander im Gewichtsverhältnis A:B = 1:2 bis 2:1 zu einem gelförmigen Färbeansatz vermischt werden, **dadurch gekennzeichnet, daß** die Zubereitung von Oxidationsfarbstoffvorprodukten (A) als Trägerkomponenten
6,0 bis 15 Gew.-% technischer Fettalkoholmischungen aus gesättigten oder ungesättigten, linearen oder verzweigten Alkoholen mit 12 bis 18 Kohlenstoffatomen, ausgewählt aus Kokos-, oder Palmkernalkohol
0,1 bis 20 Gew.-% eines niedermolekularen wasserlöslichen Alkohols,
0,1 bis 15 Gew.-% einer flüssigen Fettsäure mit 16 bis 22 C-Atomen in Form einer wasserlöslichen Seife und
0,1 bis 20 Gew.-% eines Anlagerungsproduktes von 1 bis 5 Mol Ethylenoxid an einen linearen Fettalkohol mit 8 bis 22 C-Atomen enthält,
und die Oxidationsmittelzubereitung (B)
3 bis 12 Gew.-% Wasserstoffperoxid
0,1 bis 5 Gew.-% 1 bis 5 Gew.-% eines wasserlöslichen, synthetischen Tensids und eines dispergierten Acrylsäure- und/oder Methacrylsäure-Polymerisats oder -Copolymerisats enthält.

2. Haarfärbemittel nach Anspruch 1, **dadurch gekennzeichnet, daß** es weiterhin 0,1 bis 15 Ges.-% eines Amins gemäß Formel (I), 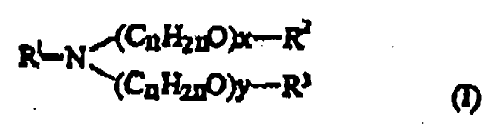 in der R¹ für einen gesättigten oder ungesättigten Alkylrest mit 8 bis 22 C-Atomen, R² und R³ unabhängig voneinander für Wasserstoff oder eine Acyl-Gruppe R⁴CO stehen, bei der wiederum R⁴ für eine C₁-C₂₁-Alkyl- oder C₂-C₂₁-Alkenylgruppe steht, n ist gleich 2 oder 3, x und y stehen für eine Zahl von 0 bis 5, mit der Maßgabe, daß die Summe aus x und y 2 bis 6 beträgt, enthält.

3. Haarfärbemittel nach einem der Ansprüche 1 oder 2, **dadurch gekennzeichnet, daß** die Zubereitung von Oxidationsfarbstoffvorprodukten (A) ein Polyol enthält.

4. Haarfärbemittel nach einem der Ansprüche 1 bis 3, **dadurch gekennzeichnet, daß** die Zubereitung von Oxidationsfarbstoffvorprodukten (A) ein wasserlösliches synthetisches Tensid, ausgewählt aus der Gruppe der kationischen, amphoteren, zwitterionischen Tenside und der Fettalkylglykoside, enthält.

5. Haarfärbemittel nach einem der Ansprüche 1 bis 4, **dadurch gekennzeichnet, daß** die Zubereitung von Oxidationsfarbstoffvorprodukten (A) Dicetylstearylether, Dicetylstearyloxyethylether, Dicetylstearyldioxyethylether und/oder N,N-Bis-(2-cetyltearyloxyethyl)-aminoethanol enthält.

6. Haarfärbemittel nach einem der Ansprüche 1 bis 5, **dadurch gekennzeichnet, daß** die Zubereitung von Oxidationsfarbstoffvorprodukten (A) eine Entwicklersubstanz ausgewählt aus der Gruppe, die gebildet wird durch 1,3-Bis-(N-(2-Hydroxyethyl)-N-(4-aminophenyl)-amino)-2-propanol, 6-Chlor-2-aminophenol, 1,10-Bis-(2,5-diaminophenyl)-1,4,7,10-tetraoxadecan, 6-Hydroxy-2,4,5-triaminopyrirnidin, 2-(2,5-Diaminophenyl)-ethanol, 2,6-Dichloraminopheilol, 3-Methyl-4-aminophenol, 2,4,5,6-Tetraaminopyrimidin, 2-Aminomethyl-4-aminophenol und 2-Hydroxymethyl-4-aminophenol, enthält.

7. Haarfärbemittel nach einem der Ansprüche 1 bis 6, **dadurch gekennzeichnet, daß** die Zubereitung von Oxidationsfarbstoffvorprodukten (A) eine Kupplersubstanz ausgewählt aus der Gruppe, die gebildet wird durch 2,4-Diaminophenoxyethanol, 2-Methylresorcin, 1,3-Bis-(2,4-diaminophenoxy)-propan, 2,7-Dihydroxynaphthalin,α-Naphthol, 2,6-Dihydroxy- 3,4-dimethylpyridin, 1,5-Dihydroxynaphthalin, 5-Amino-2-methylphenol, 2-Amino-4-(β-hydroxyethylamino)anisol, 3-Amino-2-chlor-6-methylphenol und 5-Amino-4-chlor-2-methylphenol, enthält.

8. Haarfärbemittel nach einem der Ansprüche 1 bis 7, **dadurch gekennzeichnet, daß** die Zubereitung von Oxidationsfarbstoffvorprodukten (A) ein Protein bzw. Proteinhydrolysat, ausgewählt aus der Gruppe, die gebildet wird durch Erbsen-, Soja- und Mandelproteinhydrolysate, Akazienprotein sowie Collagen- und Keratinhydrolysat, enthält.

## Claims

1. A hair colorant consisting of a flowable preparation of oxidation dye precursors (A) and a flowable aqueous oxidizing agent preparation (B) which are mixed together in a ratio by weight of A to B of 1:2 to 2:1 immediately before application to the hair to form a colouring gel, **characterized in that** the preparation of oxidation dye precursors (A) contains as carrier components
6.0 to 15% by weight of technical mixtures of saturated or unsaturated, linear or branched C₁₂₋₁₈ fatty alcohols, selected from coconut oil or palm kernel oil fatty alcohol
0.1 to 20% by weight of a low molecular weight water-soluble alcohol,
0.1 to 15% by weight of a liquid C₁₆₋₂₂ fatty acid in the form of a water-soluble soap and
0.1 to 20% by weight of an addition product of 1 to 5 mol ethylene oxide onto a linear C₈₋₂₂ fatty alcohol,
and the oxidizing agent preparation (B) contains
3 to 12% by weight of hydrogen peroxide
0.1 to 5% by weight of a water-soluble synthetic surfactant and
1 to 5% by weight of a dispersed acrylic and/or methacrylic acid polymer or copolymer.

2. A hair colorant as claimed in claim 1, **characterized in that** it additionally contains 0.1 to 15% by weight of an amine corresponding to formula (I) 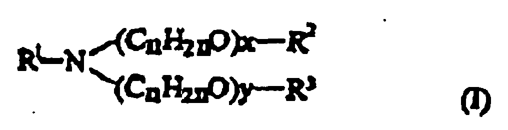 in which R¹ is a saturated or unsaturated C₈₋₂₂ alkyl group, R² and R³ independently of one another represent hydrogen or an acyl group R⁴CO where R⁴ is a C₁₋₂₁ alkyl or C₂₋₂₁ alkenyl group, n = 2 or 3, x and y are numbers of 0 to 5, with the proviso that the sum of x and y is 2 to 6.

3. A hair colorant as claimed in any of claims 1 or 2, **characterized in that** the preparation of oxidation dye precursors (A) contains a polyol.

4. A hair colorant as claimed in any of claims 1 to 3, **characterized in that** the preparation of oxidation dye precursors (A) contains a water-soluble synthetic surfactant selected from the group of cationic, amphoteric, zwitterionic surfactants and fatty alkyl glycosides.

5. A hair colorant as claimed in any of claims 1 to 4, **characterized in that** the preparation of oxidation dye precursors (A) contains dicetyl stearyl ether, dicetyl stearyl oxyethyl ether, dicetyl stearyl dioxyethyl ether and/or N,N-bis-(2-cetylstearyloxyethyl)-aminoethanol.

6. A hair colorant as claimed in any of claims 1 to 5, **characterized in that** the preparation of oxidation dye precursors (A) contains a primary intermediate selected from the group consisting of 1,3-bis-(N-(2-hydroxyethyl)-N-(4-aminophenyl)-amino)-2-propanol, 6-chloro-2-aminophenol, 1,10-bis-(2,5-diaminophenyl)-1,4,7,10-tetraoxadecane, 6-hydroxy-2,4,5-triaminopyrimidine, 2-(2,5-diaminophenyl)-ethanol, 2,6-dichloroaminophenol, 3-methyl-4-aminophenol, 2,4,5,6-tetraaminopyrimidine, 2-aminomethyl-4-aminophenol and 2-hydroxymethyl-4-aminophenol.

7. A hair colorant as claimed in any of claims 1 to 6, **characterized in that** the preparation of oxidation dye precursors (A) contains a secondary intermediate selected from the group consisting of 2,4-diaminophenoxyethanol, 2-methylresorcinol, 1,3-bis-(2,4-diaminophenoxy)-propane, 2,7-dihydroxynaphthalene, α-naphthol, 2,6-dihydroxy-3,4-dimethylpyridine, 1,5-dihydroxynaphthalene, 5-amino-2-methylphenol, 2-amino-4-(β-hydroxyethylamino)-anisole, 3-amino-2-chloro-6-methylphenol and 5-amino-4-chloro-2-methylphenol.

8. A hair colorant as claimed in any of claims 1 to 7, **characterized in that** the preparation of oxidation dye precursors (A) contains a protein or protein hydrolyzate selected from the group consisting of pea, soya and almond protein hydrolyzate, acacia protein and collagen and keratin hydrolyzate.

## Revendications

1. Teinture pour les cheveux consistant en une préparation apte à l'écoulement de produits précurseurs de colorants par oxydation (A) et en une préparation d'agent d'oxydation (B), aqueuse apte à l'écoulement qui sont mélanges l'une avec l'autre immédiatement avant l'application sur les cheveux, dans le rapport A : B = 1 : 2 à 2 : 1, en un échantillon de couleur sous forme de gel, **caractérisée en ce que** la préparation de produits précurseurs de colorant par oxydation (A) contient comme composant de support:
de 6,0 à 15% en poids de mélanges industriels de alcools gras saturés ou non saturés, linéaires ou ramifiés ayant de 12 à 18 atomes de carbone, choisi parmi l'alcool gras de coco ou de palmiste,
de 0,1 à 20 % en poids d'un alcool soluble dans l'eau, de faible poids moléculaire,
de 0,1 à 15 % en poids d'un acide gras liquide ayant de 16 à 22 atomes de carbone sous forme d'un savon soluble dans l'eau, et
de 0,1 à 20 % en poids d'un produit d'addition de 1 à 5 mol d'oxyde d'éthylène sur un alcool gras linéaire ayant de 8 à 22 atomes de carbone,
et la préparation d'agent d'oxydation (B) contient
de 3 à 12 % en poids de peroxyde d'hydrogène,
de 0,1 à 5 % en poids d'un agent tensioactif soluble dans l'eau, synthétique, et
de 1 à 5 % en poids d'un polymérisat ou d'un copolymérisat d'acide acrylique et/ou d'acide méthacrylique dispersé.

2. Teinture pour cheveux selon la revendication 1, **caractérisée en ce qu'**elle contient en outre de 0,1 à 15 % en poids d'une amine selon la formule 1 : 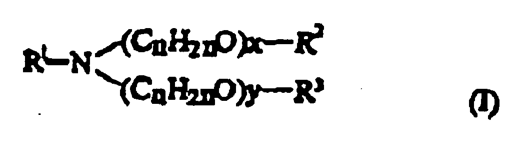 dans laquelle R¹ représente un reste alkyle saturé ou non saturé ayant de 8 à 22 atomes de carbone, R² et R³ indépendamment l'un de l'autre représentent de l'hydrogène ou un groupe acyle R⁴CO dans lequel à nouveau R⁴ représente un groupe alkyle en C₁ à C₂₁ ou un groupe alkenyle en C₂ à C₂₁, n est égal à 2 ou 3, x et y représentent un nombre allant de 0 à 5, avec la précision que la somme de x et de y vaut 2 à 6.

3. Teinture pour cheveux selon l'une quelconque des revendications 1 ou 2, **caractérisée en ce que** la préparation de produits précurseurs de colorants par oxydation (A) renferme un polyol.

4. Teinture pour cheveux selon l'une quelconque des revendications 1 à 3, **caractérisée en ce que** la préparation de produits précurseurs de colorants par oxydation (A) renferme un agent tensioactif synthétique soluble dans l'eau choisi dans le groupe des agents tensioactifs cationiques, amphotères, zwitterioniques et des (alkyl gras)glycosides.

5. Teinture pour cheveux selon l'une quelconque des revendications 1 à 4, **caractérisée en ce que** la préparation de produits précurseurs de colorant par oxydation (A) contient de l'éther dicétylstéarique, de l'éther dicétylstéaryloxyéthylique, de l'éther dicétylstéaryl-dioxyéthylique et/ou du N,N-bis(2-cétylstéaryloxyéthyl)-aminoéthanol.

6. Teinture pour cheveux selon l'une quelconque des revendications 1 à 5, **caractérisée en ce que** la préparation des produits précurseurs de colorant par oxydation (A) contient une substance d'agent de revelation choisi dans le groupe qui est formé par le 1,3-bis-(N-(2-hydroxyéthyl)-N-[4-aminophényl)-amino]-2-propanol, le 6-chloro-2-aminophénol, le 1,10-bis-(2,5-diaminophényl)-1,4,7,10-tétraoxadécane, la 6-hydroxy-2,4,5-triaminopyrimidine, le 2-(2,5-diaminophényl)-éthanol, le 2,6-dichloroaminophénol, le 3-méthyl-4-aminophénol, la 2,4,5,6-tétraaminopyrimidine, le 2-aminométhyl-4-aminophénol et le 2-hydroxyméthyl-4-aminophénol.

7. Teinture pour cheveux selon l'une quelconque des revendications 1 à 6, **caractérisée en ce que** la préparation de produits précurseurs de colorant par oxydation (A) contient une substance de couplage choisie dans le groupe formé par le 2,4-diaminophénoxyéthanol, le 2-méthylresorcinol, le 1,3-bis-(2,4-diaminophénoxyl) propane, le 2,7-dihydroxy-naphtalène, l'α-naphtol, la 2,6-dihydroxy-3,4-diméthylpyridine, le 1,5-dihydroxy-naphtalène, le 5-amino-2-méthylphénol, le 2-amino-4-(β-hydroxyéthylamino)anisol, le 3-amino-2-chloro-6-méthylphénol et le 5-amino-4-chloro-2-méthylphénol.

8. Teinture pour cheveux selon l'une quelconque des revendications 1 à 7, **caractérisée en ce que** la préparation de produits précurseurs de colorant par oxydation (A) contient une protéine ou un hydrolysat de protéine choisi dans le groupe formé par un hydrolysat de protéine de pois, de soja, et d'amandes, une protéine d'acacia ainsi qu'un hydrolysat de collagène et de kératine.
